# EUROPEAN PATENT SPECIFICATION

(11) **EP 2 123 656 B1**
(45) Date of publication and mention of the grant of the patent: **11.06.2014**
(21) Application number: 08712139.8
(22) Date of filing: 29.02.2008
(51) Int. Cl.: C07D 495/04, A61K 31/4365, A61P 7/02

(54) **PROCESS FOR PRODUCTION OF PRASUGREL HYDROCHLORIDE HAVING HIGH PURITY**
VERFAHREN ZUR HERSTELLUNG VON HOCHREINEM PRASUGRELHYDROCHLORID
PROCÉDÉ DE PRODUCTION DE CHLORHYDRATE DE PRASUGREL DE GRANDE PURETÉ

(30) Priority: 02.03.2007 JP 2007053093
(43) Date of publication of application: 25.11.2009
(73) Proprietor: Daiichi Sankyo Company, Limited, Chuo-ku Tokyo 103-8426 (JP); Ube Industries, Ltd., Ube-shi Yamaguchi 755-8633 (JP)
(72) Inventor: MIYATA, Hiroyuki, Ube-shi, Yamaguchi 755-8633 (JP); WADA, Yukinori, Ube-shi, Yamaguchi 755-8633 (JP); YOKOTA, Naoyuki, Ube-shi, Yamaguchi 755-8633 (JP)
(74) Representative: Wallace, Sheila Jane
(86) International application number: PCT/JP2008/053617
(87) International publication number: WO 2008/108291

(56) References cited:
- EP-A1- 1 298 132
- WO-A1-96/11203
- WO-A1-2007/114526
- JP-A- 06 041 139
- JP-A- 2002 145 883
- JP-A- 2003 246 735
- US-A- 5 874 581

## Description

### Technical Field

The present invention relates to a method for producing high-purity prasugrel hydrochloride.

### Background Art

The compound having the formula: 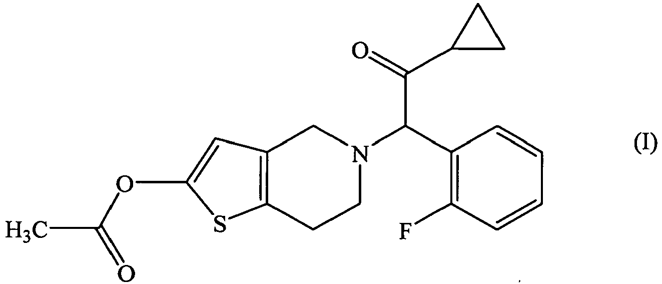 is well known as prasugrel. Prasugrel and pharmaceutically acceptable salts thereof are each known to have a platelet aggregation-inhibiting activity and are useful as an active ingredient of a medicine (particularly, an antithrombotic or anti-embolic agent) (Patent Document 1 or 2). However, the use of prasugrel or a pharmaceutically acceptable salt thereof as a medicine has required a technique for producing prasugrel or a pharmacologically acceptable salt thereof of a high purity.

Prasugrel hydrochloride represented by the formula: 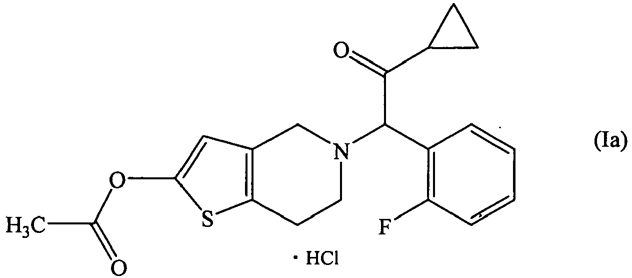 can be produced by the following production method. Patent Document 3 discloses steps (i) to (iii), and Patent Document 2 discloses step (iv). However, neither of these Patent Documents describes the by-product CATP.

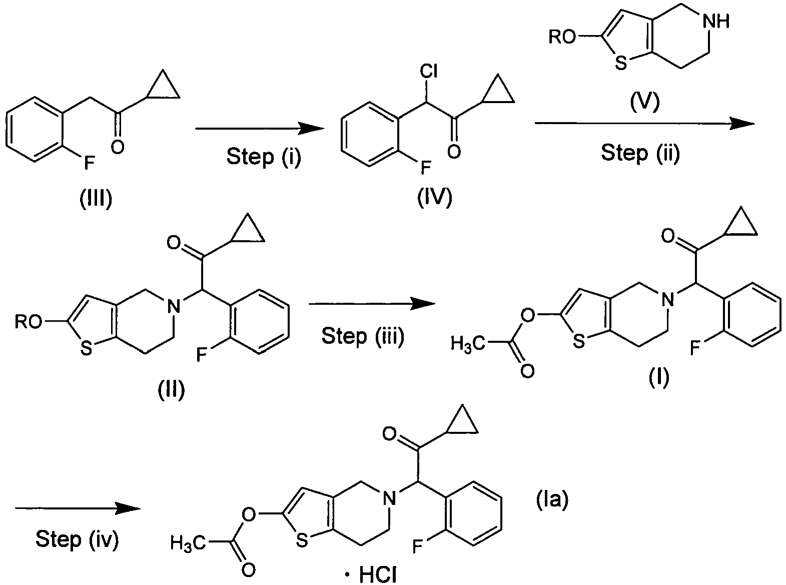

In the formulae, R represents a protecting group for the hydroxyl group.
Patent Document 1: Japanese Patent Laid-Open No. Hei 6-41139
Patent Document 2: Japanese Patent Laid-Open No. 2002-145883
Patent Document 3: International Publication No. WO96/11203

### Disclosure of the Invention

### Problems to be Solved by the Invention

The present inventors have found that the large-scale production of prasugrel hydrochloride by the above method causes the final product to be contaminated with the by-product CATP which has not previously been known.

An object of the present invention is to provide a method for producing high-purity prasugrel hydrochloride with a reduced content of by-products such as CATP.

### Means for Solving the Problems

As a result of intensive studies on a method for producing high-purity prasugrel hydrochloride with a reduced content of impurities such as the by-product CATP, the present inventors have found that the reaction temperature can be controlled in the chlorination step as step (i) of the above production method to reduce the content of the by-product CATP in prasugrel hydrochloride as the final desired compound. Thereby, the present invention has been accomplished.

For reaction conditions in the chlorination step as step (i), International Publication No. WO96/11203 describes in Reference Examples 12-1 and 12-2 that a chlorinating agent was "added dropwise while the liquid temperature was maintained to be lower than 5°C. After the liquid temperature was gradually raised to room temperature (20°C), the mixture was allowed to react under stirring for 1.5 hours". Thus, the reaction temperature after the addition, optionally dropwise, of the chlorinating agent has previously been considered to be preferably room temperature or higher. In contrast, the present invention has enabled a reduction of the content of the by-product CATP in the final desired compound prasugrel hydrochloride by controlling at low values the reaction temperature after the addition, optionally dropwise, of the chlorinating agent as well as the temperature during the addition, optionally dropwise, of the chlorinating agent.

The present invention provides a method for producing prasugrel hydrochloride, characterized by controlling the reaction temperature in step (i) of the above production steps (i) to (iv).

The present invention is:
(1) A method for producing prasugrel hydrochloride, comprising the steps of:
   (i) chlorinating compound (III) by adding a chlorinating agent thereto in a solvent;
   (ii) reacting the resultant compound (IV) with compound (V) or a salt thereof in a solvent in the presence of a base;
   (iii) acetylating the resultant compound (II) by reacting an acetylating agent therewith in a solvent in the presence of a base and an acylation catalyst; and
   (iv) adding hydrochloric acid to the resultant compound (I) in a solvent, thereby producing prasugrel hydrochloride
   characterized in that, in step (i), the temperature during the addition of the chlorinating agent is -20°C to 5°C and the reaction temperature after the addition of the chlorinating agent is -20°C to 5°C;
(2) A method as described in item (1), **characterized in that**, in step (i), the temperature during the addition of the chlorinating agent is -10°C to 5°C and the reaction temperature after the addition of the chlorinating agent is -10°C to 5°C;
(3) A method as described in item (1), **characterized in that**, in step (i), the temperature during the addition of the chlorinating agent is -5°C to 5°C and the reaction temperature after the addition of the chlorinating agent is -5°C to 5°C;
(4) A method as described in any one of items (1) to (3), characterised in that the chlorinating agent is added dropwise,
(5) A method as described in any one of items (1) to (4), characterised in that the hydrochloric acid is added dropwise;
(6) A method as described in any one of items (1) to (5), characterized in that the temperature of post-treatment after the end of the reaction in step (i) is -20°C to 15°C;
(7) A method as described in any one of items (1) to (5), characterized in that the temperature of post-treatment after the end of the reaction in step (i) is -10°C to 15°C;
(8) A method as described in any one of items (1) to (5), characterized in that the temperature of post-treatment after the end of the reaction in step (i) is0°C to 15°C;
(9) A method as described in any one of items (1) to (5), wherein the chlorinating agent is chloride gas;
(10) A method as described in any one of items (1) to (9), wherein R is a group represented by the general formula; 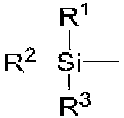 wherein R¹, R² and R³ independently represent an alkyl group having 1 to 10 carbons or an aryl group;
(11) A method as described in item (10), wherein R¹, R² and R³ independently represent an alkyl group having 1 to 5 carbons or a phenyl group;
(12) A method as described in any one of items (1) to (9), wherein R is a tert-butyldimethylsilyl group;
(13) A method as described in any one of items (1) to (12)characterized in that the resultant compound (II) is recrystallized from ethers or nitriles in step (ii);
(14) A method as described in any one of items (1) to (12), characterized in that the resultant compound (II) is recrystallized from acetonitrile in step (ii);
(15) A method as described in any one of items (1) to (14), wherein the acetylating agent is acetic anhydride;
(16) A method as described in any one of items (1) to (15), characterized in that the resultant compound (1) obtained in step (iii) is used in the next step (iv) without purification;

According to the present invention, the "protecting group for a hydroxyl group" is not particularly limited provided that it can stably protect the hydroxyl group in the reaction, and specifically refers to a protecting group capable of being cleaved by a chemical step such as hydrogenolysis, hydrolysis, electrolysis and photolysis. The protecting group may be, for example, an aliphatic acyl group including an alkanoyl group such as a formyl, acetyl, propionyl, butyryl, isobutyryl, pentanoyl, pivaloyl, valeryl, isovaleryl, octanoyl, nonanoyl, decanoyl, 3-methylnonanoyl, 8-methylnonanoyl, 3-ethyloctanoyl, 3,7-dimethyloctanoyl, undecanoyl, dodecanoyl, tridecanoyl, tetradecanoyl, pentadecanoyl, hexadecanoyl, 1-methylpentadecanoyl, 14-methylpentadecanoyl, 13,13-dimethyltetradecanoyl, heptadecanoyl, 15-methylhexadecanoyl, octadecanoyl, 1-methylheptadecanoyl, nonadecanoyl, eicosanoyl or henaicosanoyl group, an alkylcarbonyl group substituted with a carboxy group, such as a succinoyl, glutaroyl or adipoyl group, an alkylcarbonyl group substituted with a halogen atom(s), such as a chloroacetyl, dichloroacetyl, trichloroacetyl or trifluoroacetyl group, a saturated cyclic hydrocarbon-carbonyl group such as a cyclopropylcarbonyl, cyclobutylcarbonyl, cyclopentylcarbonyl, cyclohexylcarbonyl, cycloheptylcarbonyl or cyclooctylcarbonyl group, an alkylcarbonyl group substituted with a lower alkoxy group, such as a methoxyacetyl group, or an unsaturated alkylcarbonyl group such as a (E)-2-methyl-2-butenoyl group; an aromatic acyl group including an arylcarbonyl group such as a benzoyl, α-naphthoyl, β-naphthoyl, pyridoyl, thienoyl or furoyl group, a halogenoarylcarbonyl group such as a 2-bromobenzoyl or 4-chlorobenzoyl group, an arylcarbonyl group substituted with a lower alkyl group(s), such as a 2,4,6-trimethylbenzoyl or 4-toluoyl group, a lower alkoxylated arylcarbonyl group such as a 4-anisoyl group, an arylcarbonyl group substituted with a carboxy group, such as a 2-carboxybenzoyl, 3-carboxybenzoyl or 4-carboxybenzoyl group, a nitrated arylcarbonyl group such as a 4-nitrobenzoyl or 2-nitrobenzoyl group, an arylcarbonyl group substituted with a lower alkoxycarbonyl, such as a 2-(methoxycarbonyl)benzoyl group, or an arylcarbonyl group substituted with an aryl, such as a 4-phenylbenzoyl group; a carbonyloxyalkyl group including a oxodioxolenylmethyl group such as a (5-methyl-2-oxo-1,3-dioxolen-4-yl)methyl or (5-phenyl-2-oxo-1,3-dioxolen-4-yl)methyl group; a half ester salt residue of succinic acid; an ester salt residue of phosphoric acid; an ester-forming residue such as an amino acid; a carbamoyl group; a carbamoyl group substituted with one or two lower alkyl groups; a carbonyloxyalkyloxycarbonyl group such as a pivaloyloxymethyloxycarbonyl group; or a silyl group such as a trimethylsilyl, triethylsilyl, tripropylsilyl, triisopropylsilyl, tert-butyldimethylsilyl or tert-butyldiphenylsilyl group. Among these protecting groups, silyl groups are preferred; more preferred is a group represented by the general formula: 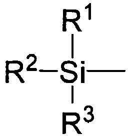 wherein R¹, R² and R³ independently represent an alkyl group having 1 to 10 carbons or an aryl group and are preferably independently an alkyl group having 1 to 5 carbons or a phenyl group; and still more preferred is a tert-butyldimethylsilyl group.

According to the present invention, the "alkyl group having 1 to 10 carbons" may be a straight-chain or branched alkyl group having 1 to 10 carbons, such as, for example, a methyl group, an ethyl group, a propyl group (including an isomer thereof), a butyl group (including each isomer thereof), a pentyl group (including each isomer thereof), a hexyl group (including each isomer thereof), a heptyl group (including each isomer thereof), an octyl group (including each isomer thereof), a nonyl group (including each isomer thereof) or a decyl group (including each isomer thereof). Preferably, it is an alkyl group having 1 to 5 carbons; more preferably a methyl group, an ethyl group, a propyl group (including an isomer thereof) or a butyl group (including each isomer thereof); and still more preferably a methyl group or a tert-butyl group.

According to the present invention, the "aryl group" is, for example, a phenyl group, a tolyl group, a xylyl group, a biphenyl group, a naphthyl group, an anthryl group, or a phenanthryl group, and is preferably an aryl group having 6 to 8 carbons, more preferably a phenyl group.

The compound produced by the method of the present invention may have an asymmetric carbon in the molecule; there may be optical isomers (including diastereomers) based thereon, which are also encompassed by the compound produced by the method of the present invention.

According to the present invention, a salt of the compound (V) may be, for example, a mineral acid salt such as a hydrochloride or sulfate; an organic sulfonate such as a p-toluenesulfonate or methanesulfonate; or an organic carboxylate such as an acetate or propionate. Mineral acid salts or organic sulfonates are preferred and a hydrochloride or a p-toluenesulfonate is more preferred.

According to the present invention, "CATP" is 2-acetoxy-5-[5-chloro-1-(2-fluorophenyl)-2-oxopentyl]-4,5,6,7-tetrahydrothieno[3,2-c]pyridine represented by the formula:

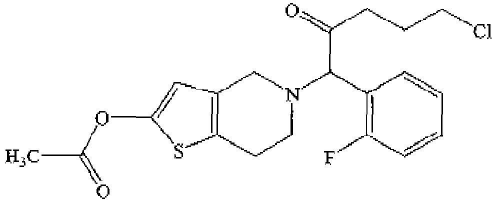

There is an asymmetric carbon in CATP according to the present invention and optical isomers can be present based thereon; any of the isomers and mixtures thereof are also encompassed by CATP according to the present invention.

### Effect of the Invention

According to the present invention, there can be provided a method for producing high-purity prasugrel hydrochloride with a reduced content of impurities such as the by-product CATP. In particular, the present invention enables the by-product CATP to be greatly reduced compared to other structurally similar by-products.

### Brief Description of the Drawings

Figure 1 is a liquid chromatography of the prasugrel hydrochloride obtained in Example 1;
Figure 2 is a liquid chromatography of the prasugrel hydrochloride obtained in Example 2; and
Figure 3 is a liquid chromatography of the prasugrel hydrochloride obtained in Reference Example 1.

### Best Mode for Carrying Out the Invention

Compound (III) used as the starting material in step (i) of the present invention can be produced by the method described in International Publication No. WO96/11203.

Compound (V) used as the starting material in step (ii) of the present invention can be produced by the method described, for example, in International Publication No. WO96/11203.

A method embodying the present invention to produce high-purity prasugrel hydrochloride is as follows.

### Step (i)

This step is a step which involves chlorinating compound (III) by adding a chlorinating agent optionally dropwise thereto in a solvent to produce compound (IV).

The chlorinating agent used in this step may be, for example, chlorine gas or sulfuryl chloride and is preferably chlorine gas.

The solvent used in this step is not particularly limited provided that it dissolves the starting material to some extent and does not inhibit the reaction. The solvent may be, for example, an ether solvent such as tetrahydrofuran, diethyl ether or dioxane; a halogenated solvent such as dichloromethane or 1,2-dichloroethane; an aromatic hydrocarbon solvent such as benzene, toluene or xylene; a nitrile solvent such as acetonitrile, propionitrile or benzonitrile; or an amide solvent such as dimethylformamide, dimethylacetamide or dimethylimidazolidone. Halogen solvents are preferred and dichloromethane is more preferred.

The amount of the chlorinating agent used in this step is typically 0.5 to 3 moles, preferably 0.8 to 2 moles, more preferably 0.9 to 1.5 moles based on 1 mole of compound (III).

When the chlorinating agent is added, optionally dropwise, in this step, the temperature of the reaction solution varies depending on the reagent, solvent, or the like; however, it is typically -20°C to 5°C, preferably -10°C to 5°C, more preferably -5°C to 5°C.

The time to add the chlorinating agent optionally dropwise in this step varies depending on the type and amount of the chlorinating agent. However, it is typically 30 minutes to 24 hours, preferably 1 hour to 12 hours, more preferably 1 hour to 6 hours.

The reaction temperature after the addition optionally dropwise of the chlorinating agent in this step varies depending on the reagent, solvent, or the like. However, it is typically -20°C to 5°C, preferably -10°C to 5°C, more preferably -5°C to 5°C.

The reaction time after the addition optionally dropwise of the chlorinating agent in this step varies depending on the reagent, solvent, reaction temperature, or the like. However, it is typically 30 minutes to 12 hours, preferably 1 hour to 6 hours, more preferably 1 hour to 3 hours.

After completion of the reaction in this step, compound (IV) may be isolated by a technique commonly used in the field of organic synthetic chemistry. The reaction liquid may also be directly used in the next step (ii) without isolation of compound (IV).

The temperature of post-treatment after the end of the reaction in this step is typically -20°C to 15°C, preferably -10°C to 15°C, more preferably 0°C to 15°C.

### Step (ii)

This step is a step which involves producing compound (II) by reacting compound (IV) with compound (V) or a salt thereof in a solvent in the presence of a base.

The amount of compound (IV) in this step is typically 0.5 to 3 moles, preferably 0.8 to 2 moles, more preferably 0.9 to 1.2 moles based on 1 mole of compound (V).

The solvent used in this step is not particularly limited provided that it dissolves the starting material to some extent and does not inhibit the reaction. The solvent may be, for example, an ether solvent such as tetrahydrofuran, diethyl ether or dioxane; a halogenated solvent such as dichloromethane or 1,2-dichloroethane; an aromatic hydrocarbon solvent such as benzene, toluene or xylene; a nitrile solvent such as acetonitrile, propionitrile or benzonitrile; or an amide solvent such as dimethylformamide, dimethylacetamide or dimethylimidazolidone. Ether solvents, halogenated solvents, nitrile solvents, or amide solvents are preferred and tetrahydrofuran, dichloromethane, acetonitrile, or dimethylacetamide is more preferred.

The base used in this step is not particularly limited. Tertiary amines are preferred, for example, trialkyl monoamines such as triethylamine, tributylamine or diisopropylethylamine; or trialkyl diamines such as diazabicyclooctane, diazabicycloundecene or tetramethylethyldiamine, more preferably trialkyl monoamines, still more preferably triethylamine, tributylamine or diisopropylethylamine.

The amount of the base used in this step is typically 0.5 to 3 moles, preferably 0.5 to 2 moles, more preferably 0.7 to 1.5 moles based on 1 mole of the compound (V).

In this step, a reaction-promoting effect is expected by allowing an ammonium salt or a quaternary ammonium salt to be present in the reaction system.

The reaction-promoting additive may be, for example, quaternary ammonium salts including tetraalkylammonium halides having alkyl groups having 1 to 20 carbons, such as tetramethylammonium chloride, tetramethylammonium bromide, tetraethylammonium chloride, tetraethylammonium bromide, tetrabutylammonium chloride or tetrabutylammonium bromide, or trialkylmonobenzylammonium halides having alkyl groups having 1 to 20 carbons, such as trimethylbenzylammonium chloride or triethylbenzylammonium chloride; alkali metal bromides including lithium bromide, sodium bromide, potassium bromide, or caesium bromide; or alkali metal iodides including lithium iodide, sodium iodide, potassium iodide, or caesium iodide. Tetraethylammonium bromide, tetrabutylammonium bromide, or sodium iodide is preferred.

The amount of the reaction-promoting additive used in this step is typically 0.01 to 5 moles, preferably 0.1 to 2 moles, based on 1 mole of compound (VI) for the quaternary ammonium salts and typically 0.001 to 0.6 mole, preferably 0.01 to 0.5 mole, based on 1 mole of compound (VI) for the alkali metal bromides or alkali metal iodides.

The reaction temperature in this step varies depending on the reagent, solvent, or the like. However, it is typically -20°C to 100°C, preferably -10°C to 70°C, more preferably 0°C to 60°C.

The reaction time in this step varies depending on the reagent, solvent, reaction temperature, or the like. However, it is typically 30 minutes to 24 hours, preferably 1 hour to 12 hours, more preferably 1 hour to 10 hours.

After completion of the reaction in this step, compound (II) may be isolated by a technique commonly used in the field of organic synthetic chemistry. The reaction liquid may also be directly used in the next step (iii) without isolation of compound (II). However, it is preferred that compound (II) is isolated and purified by recrystallization. This further reduces the content of the by-product CATP in prasugrel hydrochloride as the final product of the present invention, which can be expected to provide higher-purity prasugrel hydrochloride.

The solvent used for the recrystallization of compound (II) is not particularly limited provided that it dissolves compound (II) to some extent and does not react with compound (II). The solvent may be, for example, an ether solvent such as tetrahydrofuran, diethyl ether or dioxane; a halogenated solvent such as dichloromethane or 1,2-dichloroethane; an aromatic hydrocarbon solvent such as benzene, toluene or xylene; a nitrile solvent such as acetonitrile, propionitrile or benzonitrile; or an amide solvent such as dimethylformamide, dimethylacetamide or dimethylimidazolidone. Ether solvents or nitrile solvents are preferred and acetonitrile is more preferred.

The temperature during the recrystallization is typically 30°C to 80°C, preferably 40°C to 70°C, more preferably 40°C to 60°C. After dissolution, the solution is gradually cooled. It is preferred that a poor solvent (preferably water) is added thereto at 30°C, which is then cooled to -5°C to 10°C and stirred for 1 hour to 6 hours. A seed crystal may also be added as needed.

### Step (iii)

This step is a step which involves acetylating compound (II) by reacting an acetylating agent therewith in a solvent in the presence of a base and an acylation catalyst to produce compound (I).

The acylation catalyst used in this step may be, for example, a 4-dialkylaminopyridine such as 4-dimethylaminopyridine, 4-diethylaminopyridine or 4-dipropylaminopyridine, and is preferably 4-dimethylaminopyridine.

The amount of the acylation catalyst used in this step is typically 0.1 to 10 mole% based on 1 mole of compound (II) and may be used in an excess amount.

The acetylating agent used in this step may be, for example, acetic anhydride or acetyl chloride, and is preferably acetic anhydride.

The amount of acetic anhydride used in this step is typically 1 to 10 moles, preferably 1 to 5 moles, based on 1 mole of compound (II).

The solvent used in this step is not particularly limited provided that it dissolves the starting material to some extent and does not inhibit the reaction. The solvent may be, for example, an ether solvent such as tetrahydrofuran, diethyl ether or dioxane; a halogenated solvent such as dichloromethane or 1,2-dichloroethane; an aromatic hydrocarbon solvent such as benzene, toluene or xylene; a nitrile solvent such as acetonitrile, propionitrile or benzonitrile; or an amide solvent such as dimethylformamide, dimethylacetamide or dimethylimidazolidone. Ether solvents, halogenated solvents, nitrile solvents, or amide solvents are preferred and tetrahydrofuran, dichloromethane, acetonitrile, or dimethylacetamide is more preferred.

The base used in this step is not particularly limited. Tertiary amines are preferred, for example, a trialkyl monoamine such as triethylamine, tributylamine or diisopropylethylamine, or a trialkyl diamine such as diazabicyclooctane, diazabicycloundecene or tetramethylethyldiamine, more preferably a trialkyl monoamine, still more preferably triethylamine.

The amount of the base used in this step is typically 1 to 10 moles, preferably 1 to 5 moles, based on 1 mole of compound (II).

The reaction temperature in this step varies depending on the reagent, solvent, or the like. However, it is typically -50°C to 50°C, preferably -30°C to 30°C, more preferably -20°C to 20°C.

The reaction time in this step varies depending on the reagent, solvent, reaction temperature, or the like. However, it is typically 30 minutes to 24 hours, preferably 1 hour to 12 hours, more preferably 1 hour to 6 hours.

After completion of the reaction in this step, compound (I) may be isolated by a technique commonly used in the field of organic synthetic chemistry. The reaction liquid may also be directly used in the next step (iv) without isolation of compound (I).

### Step (iv)

This step is a step which involves producing prasugrel hydrochloride by adding hydrochloric acid optionally dropwise to compound (I) in a solvent.

In this step, the adding of the hydrochloric acid optionally dropwise may be carried out by adding the acid dropwise or adding it at one time or in two to several divided portions.

The solvent used in this step is not particularly limited provided that it dissolves the starting material to some extent and does not inhibit the reaction. The solvent may be, for example, an aliphatic hydrocarbon such as hexane, cyclohexane, heptane or ligroin, or petroleum ether; an aromatic hydrocarbon such as benzene, toluene or xylene; a halogenated hydrocarbon such as dichloromethane, chloroform, carbon tetrachloride, 1,2-dichloroethane, chlorobenzene or dichlorobenzene; an ether such as diethyl ether, diisopropyl ether, tetrahydrofuran, dioxane, dimethoxyethane or diethylene glycol dimethyl ether; a ketone such as acetone, methyl ethyl ketone or diethyl ketone; an ester such as ethyl acetate, propyl acetate or butyl acetate; a carboxylic acid such as acetic acid or propionic acid; or a nitrile such as acetonitrile or propionitrile. Ethers, ketones, esters, carboxylic acids, or nitriles are preferred; tetrahydrofuran, dioxane, acetone, methyl ethyl ketone, ethyl acetate, acetic acid, or acetonitrile is more preferred; tetrahydrofuran, dioxane, acetic acid or acetone is still more preferred; and acetone is most preferred.

The reaction temperature in this step varies depending on the reagent, solvent, or the like. However, it is typically -20°C to 100°C, preferably 0°C to 70°C, more preferably 30°C to 60°C, most preferably 40°C to 55°C.

The reaction time in this step varies depending on the reagent, solvent, reaction temperature, or the like. However, it is typically 5 minutes to 10 hours, preferably 10 minutes to 5 hours.

A preferred embodiment of the step is a method which involves dissolving compound (I) in acetone, dropwise addition of half the necessary amount (typically, the necessary amount is equimolar to the thienopyridine form) of concentrated hydrochloric acid to the solution at 0°C to 70°C (preferably 35°C to 60°C) over a period of 2 minutes to 10 minutes, adding a seed crystal as needed, followed by reaction at the same temperature for 30 minutes to 2 hours, and further dropwise addition of the remaining necessary amount of concentrated hydrochloric acid over a period of 30 minutes to 2 hours, followed by reaction at 0°C to 70°C (preferably 25°C to 55°C) for 1 hour to 3 hours.

After completion of the reaction in this step, prasugrel hydrochloride of the present invention is collected from the reaction mixture according to a conventional method. For example, the desired compound is obtained by collecting the precipitated crystal by filtration after completion of the reaction or distilling off the solvent after completion of the reaction. The desired compound obtained may be, if necessary, further purified by a conventional method, for example, recrystallization, reprecipitation, or chromatography

Prasugrel hydrochloride produced by the method of the present invention may be allowed to stand in the air or recrystallized to absorb water, thereby having an adsorbed water or becoming a hydrate; the water-containing compound is also encompassed by a prasugrel hydrochloride produced by the method of the present invention. In addition, a solvate thereof containing any amount of a solvent is also encompassed by a prasugrel hydrochloride produced by the method of the present invention.

The content of CATP in the prasugrel hydrochloride is measured by liquid chromatography and expressed in percentage by area (%) in terms of the content of CATP in free prasugrel.

The content of CATP in the high-purity prasugrel hydrochloride produced by the method of the present invention is typically 0.3% or less, preferably 0.1% or less, more preferably 0.04% or less, still more preferably 0.03% or less, particularly preferably 0.02% or less.

The purity of the prasugrel hydrochloride, that is, the prasugrel content, can be measured as described for the CATP content.

The purity of a high-purity prasugrel hydrochloride produced by the method according to the present invention is typically 95% or more, preferably 97% or more, more preferably 99% or more.

High-purity prasugrel hydrochloride obtained by the method of the present invention is excellent in oral absorbability and metabolism-activating and platelet aggregation-inhibiting activity and weak in toxicity and further has good storage and handling stability, and therefore is useful as a medicine (preferably a prophylactic or therapeutic agent for diseases caused by thrombus or embolus (particularly, a therapeutic agent), more preferably a prophylactic or therapeutic agent for thrombosis or embolism (particularly, a therapeutic agent)). In addition, the medicine is preferably for use in warm-blooded animals, more preferably for use in humans.

When used as a therapeutic or prophylactic agent for the diseases, a high-purity prasugrel hydrochlorideproduced by the method of the present invention can be administered per se or in a proper mixture with a pharmaceutically acceptable excipient, diluent, or the like orally in the form of tablets, capsules, granules, powders, syrups, or the like or parenterally in the form of injections, suppositories, or the like.

These formulations are produced by well-known methods using additives including fillers (which may be, for example, organic fillers (e.g., sugar derivatives such as lactose, sucrose, glucose, mannitol, or sorbitol; starch derivatives such as corn starch, potato starch, α-starch, or dextrin; cellulose derivatives such as crystalline cellulose; gum Arabic; dextran; or pullulan); or inorganic fillers (e.g., silicate derivatives such as light anhydrous silicic acid, synthetic aluminum silicate, calcium silicate, or magnesium metasilicate aluminate; phosphates such as calcium hydrogenphosphate; carbonates such as calcium carbonate; or sulfates such as calcium sulfate.)), lubricants (which may be, for example, stearic acid; stearic acid metal salts such as calcium stearate or magnesium stearate; talc; waxes such as beeswax or spermaceti; boric acid; adipic acid; sulfates such as sodium sulfate; glycol; fumaric acid; sodium benzoate; D,L-leucine; laurylsulfates such as sodium lauryl sulfate or magnesium lauryl sulfate; silicates such as silicic anhydride or hydrated silicate; or a starch derivative as defined above), binders (which may be, for example, hydroxypropyl cellulose, hydroxypropyl methyl cellulose, polyvinylpyrrolidone, macrogol, or compounds similar to an excipient as defined above), disintegrators (which may be, for example, cellulose derivatives such as low-substituted hydroxypropyl cellulose, carboxymethyl cellulose calcium, or internally cross-linked carboxymethyl cellulose sodium; chemically modified starches/celluloses such as carboxymethyl starch, sodium carboxymethyl starch, or cross-linked polyvinylpyrrolidone; or a starch derivative as defined above), emulsifiers (which may be, for example, colloidal clays such as bentonite or veegum; metal hydroxides such as magnesium hydroxide or aluminum hydroxide; anionic surfactants such as sodium lauryl sulfate or calcium stearate; cationic surfactants such as benzalkonium chloride; or nonionic surfactants such as polyoxyethylene alkyl ether, polyoxyethylene sorbitan fatty acid ester, or a sucrose fatty acid ester), stabilizers (which may be, for example, para-hydroxybenzoic esters such as methylparaben or propylparaben; alcohols such as chlorobutanol, benzyl alcohol, or phenylethyl alcohol; benzalkonium chloride; phenols such as phenol, or cresol; thimerosal; dehydroacetic acid; or sorbic acid), corrigents (which may be, for example, commonly used sweeteners, acidulants or flavorings), and diluents.

The amount of use thereof may vary depending on symptoms, age, and the like, and it may be administered to an adult human once to seven times a day orally at a lower limit of 0.1 mg (preferably 1 mg) per dose and an upper limit of 1,000 mg (preferably 500 mg) per dose or intravenously at a lower limit of 0.01 mg (preferably 0.1 mg) per dose and an upper limit of 500 mg (preferably 250 mg) per dose, depending on the symptoms. Thus, the amount used per dose in a person 60 kg in weight is 0.0017 mg/kg (preferably 0.017 mg/kg) as a lower limit and 17 mg/kg (preferably 8.3 mg/kg) as an upper limit for oral administration and 0.00017 mg/kg (preferably 0.0017 mg/kg) as a lower limit and 8.3 mg/kg (preferably 4.2 mg/kg) as an upper limit for intravenous administration.

### Examples

The present invention is described below in further detail with reference to Examples, Reference Examples, and a Test Example. However, the invention is not intended to be limited thereto.

### Example 1

### (1) 2-Fluoro-α-cyclopropylcarbonylbenzyl chloride (step (i))

A mixture of 100 g of cyclopropyl 2-fluorobenzyl ketone and 886 g of dichloromethane was stirred while cooling with ice to provide a mixed solution. Into the resultant mixed solution was blown 3.98 g (0.1 equivalent) of chlorine gas over a period of 20 minutes while the solution temperature was maintained to be not higher than 5°C, which was then stirred for 0.5 hour at the same temperature. Further, 39.8 g (1 equivalent) of chlorine gas was blown thereinto over a period of 220 minutes at the same temperature, which was reacted by stirring for one hour at the same temperature.

After completion of the reaction, 236 g of a 3% sodium thiosulfate aqueous solution was added dropwise to the resultant reaction solution under stirring while the solution temperature was maintained to be not higher than 15°C. After the dropwise addition, the solution was stirred for 10 minutes and then subjected to a liquid-separating operation. The resultant organic layer was washed sequentially with 589 g of a precooled 8% sodium hydrogencarbonate aqueous solution and 168 g of precooled water and then concentrated under reduced pressure to provide 145 g of the title compound (pure content: 95.4 g, yield: 80%) in an oily form. During these operations, the solution temperature was kept at 0°C to 15°C.

### (2) 2-(tert-Butyldimethylsilyloxy)-5-(α-cyclopropylcarbonyl-2-fluorobenzyl)-4,5,6,7-tetrahydrothieno[3,2-c]pyridine (step (ii))

To a mixture of 115 g of 5,6,7,7a-tetrahydro-4H-thieno[3,2-c]pyridin-2-one p-toluenesulfonate, 60.7 g of tert-butyldimethylchlorosilane, and 277 g of dichloromethane was added 40.7 g of triethylamine, which was then stirred at 25°C for one hour to provide a mixed solution. To the mixed solution were added 78.1 g of the 2-fluoro-α-cyclopropylcarbonylbenzyl chloride obtained in (1), 70.8 g of triethylamine, and 1.57 g of sodium iodide, which were then reacted by stirring at 45°C for one hour and further at 52°C for 5 hours.

After completion of the reaction, to the resultant reaction solution was added a total amount of the phosphate buffer solution prepared by adding distilled water to 9.50 g of KH₂PO₄ and 0.95 g of Na₂HPO₄·12H₂O into a total weight of 358 g, which was then subjected to liquid-separating operation, followed by subjecting the aqueous layer to back extraction with 116 g of dichloromethane. The resultant organic layers were combined and concentrated under reduced pressure until the residue reached a volume of 218 mL. Thereto was added 476 g of acetonitrile, and the resultant mixture was then concentrated under reduced pressure until the residue reached a volume of 517 mL. To the resultant residue was added 238 g of acetonitrile, which was then stirred at 30°C for 30 minutes. Subsequently, 122 g of water was added thereto, which was then stirred at 0°C for 3 hours. The precipitated crystal was collected by filtration, washed with 69.0 g of precooled acetonitrile, and dried under reduced pressure to provide 131 g of the title compound.

### (3) 2-Acetoxy-5-(α-cyclopropylcarbonyl-2-fluorobenzyl)-4,5,6,7-tetrahydrothieno[3,2-c]pyridine (step (iii))

To a mixture of 15.0 g of 2-(tert-butyldimethylsilyloxy)-5-(α-cyclopropylcarbonyl-2-fluorobenzyl)-4,5,6,7-tetrahydrothieno[3,2-c]pyridine obtained in (2), 5.10 g of triethylamine, 41.3 mg of 4-dimethylaminopyridine, and 75 g of acetonitrile was added dropwise 3.90 g of an acetonitrile solution in which 4.13 g of acetic anhydride was dissolved, and the resultant mixture was reacted by stirring at 0°C for one hour.

After completion of the reaction, 50.6 g of cold water was added to the resultant reaction solution, which was then stirred at -15°C for 30 minutes. The precipitated crystal was collected by filtration, washed with a mixed solution of 15.1 g of acetonitrile and 11.9 g of water, and then dried under reduced pressure to provide 10.8 g of the title compound.
Melting point: 122 to 124°C.

### (4) 2-Acetoxy-5-(α-cyclopropylcarbonyl-2-fluorobenzyl)-4,5,6,7-tetrahydrothieno[3,2-c]pyridine hydrochloride (step (iv))

To 8.00 g of 2-acetoxy-5-(α-cyclopropylcarbonyl-2-fluorobenzyl)-4,5,6,7-tetrahydrothieno[3,2-c]pyridine obtained in (3) and 398 mg of activated clay was added 43 g of acetone, and the resultant mixture was then stirred at 32°C. The reaction solution was filtered, the residue was washed with 4.41 g of acetone, and then 1.12 g of 36% concentrated hydrochloric acid was added dropwise to the solution at 52°C over a period of one minute. Thereto was added as a seed crystal 238 mg of crystal B2 obtained by the method described in Japanese Patent Laid-Open No. 2002-145883, which was then stirred at the same temperature for one hour. In addition, 1.07 g of 36% concentrated hydrochloric acid was added dropwise thereto over a period of one hour, which was then stirred at 40°C for 2 hours and further at 30°C for 1 hour. The precipitated crystal was collected by filtration, washed with 15.8 g of acetone, and dried under reduced pressure at 50°C for 5 hours to provide 8.03 g of the title compound.
Melting point: 194 to 197°C.

### Example 2

### (1) 2-(tert-Butyldimethylsilyloxy)-5-(α-cyclopropylcarbonyl-2-fluorobenzyl)-4,5,6,7-tetrahydrothieno[3,2-c]pyridine (step (ii))

To 40.0 g of the compound (II) not subjected to a recrystallization operation, obtained in Example 1-(2) was added 252 g of acetonitrile, which was then stirred at 50°C for 10 minutes and cooled to 30°C. Subsequently, 40 g of water was added dropwise thereto at the same temperature over a period of 30 minutes, which was then cooled to 0°C and stirred at the same temperature for 3 hours. The precipitated crystal was collected by filtration, washed with 30 g of precooled acetonitrile, and dried under reduced pressure to provide 37.6 g of the title compound.

### (2) 2-Acetoxy-5-(a-cyclopropylcarbonyl-2-fluorobenzyl)-4,5,6,7-tetrahydrothieno[3,2-c]pyridine (step (iii))

Using 22.5 g of 2-(tert-butyldimethylsilyloxy)-5-(α-cyclopropylcarbonyl-2-fluorobenzyl)-4,5,6,7-tetrahydrothieno[3,2-c]pyridine obtained in (1), reaction and post-treatment were performed according to Example 1 (3) to provide 16.4 g of the title compound.
Melting point: 122 to 124°C.

### (3) 2-Acetoxy-5-(α-cyclopropylcarbonyl-2-fluorobenzyl)-4,5,6,7-tetrahydrothieno[3,2-c]pyridine hydrochloride (step (iv))

Using 8.00 g of 2-acetoxy-5-(α-cyclopropylcarbonyl-2-fluorobenzyl)-4,5,6,7-tetrahydrothieno[3,2-c]pyridine obtained in (2), reaction and post-treatment were performed according to Example 1 (4) to provide 8.01 g of the title compound.
Melting point: 192 to 196°C.

### Reference Example 1

### (1) 2-Fluoro-α-cyclopropylcarbonylbenzyl chloride (step (i))

A mixture of 100 g of cyclopropyl 2-fluorobenzyl ketone and 886 g of dichloromethane was stirred while cooling with ice to provide a mixed solution. Into the resultant mixed solution was blown 3.98 g (0.1 equivalent) of chlorine gas over a period of 20 minutes while the solution temperature was maintained to be not higher than 5°C, which was then stirred for 0.5 hour at the same temperature. In addition, 39.8 g (1 equivalent) of chlorine gas was blown thereinto at the same temperature over a period of 220 minutes, and the solution temperature was then gradually raised to 20°C, followed by stirring for one hour for reaction.

After completion of the reaction, 500 mL of precooled water was added dropwise to the resultant reaction solution while stirring, which was then stirred for 10 minutes and subjected to a liquid-separating operation. The resultant organic layer was washed sequentially with 833 mL of a saturated sodium hydrogencarbonate aqueous solution and 333 mL of water and then concentrated under reduced pressure to provide 129 g of the title compound (pure content: 96.1 g, yield: 81 %) in an oily form.

### (2) 2-Acetoxy-5-(α-cyclopropylcarbonyl-2-fluorobenzyl)-4,5,6,7-tetrahydrothieno[3,2-c]pyridine hydrochloride (steps (ii) to (iv))

Using 105 g (pure content: 78.3 g) of 2-fluoro-α-cyclopropylcarbonylbenzyl chloride obtained in (1), reaction and post-treatment were performed according to each of Examples 1 (2) to (4) to provide 8.10 g of the title compound.
Melting point: 194 to 196°C.

### Reference Example 2: (Production of Impurity CATP Standard)

### (1) 5-Chloro-1-(2-fluorophenyl)pentan-2-one

To 5.00 g of cyclopropyl 2-fluorobenzyl ketone was added 25 mL of 36% concentrated hydrochloric acid, which was then stirred at 100°C for 2.5 hours. After the completion of reaction, the reaction solution was cooled, to which 50 mL of water and 50 mL of dichloromethane were then added for the liquid-separating operation. The resultant organic layer was washed with 50 mL of a saturated sodium hydrogencarbonate aqueous solution, dried with anhydrous magnesium sulfate, and then concentrated under reduced pressure to provide 6.70 g of the title compound in an oily form.

### (2) 1,5-Dichloro-1-(2-fluorophenyl)pentan-2-one

To 9.44 g of 5-chloro-1-(2-fluorophenyl)pentan-2-one obtained as described in (1) was added 63 mL of dichloromethane, into which 119 mL of chlorine gas was then blown over a period of one minute while the solution temperature was maintained at 15°C, followed by stirring at the same temperature for 0.5 hour. In addition, 1.19 L of chlorine gas was blown thereinto at the same temperature over a period of 10 minutes, which was then stirred at the same temperature for 1.5 hours for reaction.

After completion of the reaction, 22 mL of a 3% sodium sulfite aqueous solution was added to the resultant reaction solution for the liquid-separating operation. The resultant organic layer was washed sequentially with 56 mL of an 8% sodium hydrogencarbonate aqueous solution and 16 mL of water, dried with anhydrous magnesium sulfate, and then concentrated under reduced pressure. The resultant residue was subjected to distillation under reduced pressure to provide 3.80 g of a fraction containing the desired compound (100°C to 105°C/48 Pa). In addition, the fraction was purified by silica gel column chromatography (elution solvent: n-hexane/ethyl acetate = 19/1 (V/V)) to provide 1.21 g of the title compound.

### (3) 2-Acetoxy-5-(5-chloro-1-(2-fluorophenyl)-2-oxopentyl)-4,5,6,7-tetrahydrothieno [3,2-c]pyridine

Using 1.21 g of 1,5-dichloro-1-(2-fluorophenyl)pentan-2-one obtained in (2), reaction and post-treatment were performed according to Examples 1 (2) and (3) to provide 1.71 g of a crude material containing the title compound. In addition, the material was purified by silica gel column chromatography (elution solvent: n-hexane/ethyl acetate = 5/1 → 3/1 (V/V)) to provide 0.77 g of the title compound in an oily form.
Mass spectrum (CI, m/z): 410 [M+H]⁺.
¹H-NMR spectrum (400 MHz, CDCl₃) δ ppm: 1.97-2.05 (m, 2H), 2.26 (s, 3H), 2.66-2.76 (m, 3H), 2.79 (t, J=5.4 Hz, 2H), 2.85-2.90 (m, 1H), 3.43-3.59 (m, 4H), 4.74 (s, 1H), 6.25 (s, 1H), 7.10-7.20 (m, 2H), 7.31-7.36 (m, 1H), 7.42-7.47 (m, 1H).

### Test Example 1

### (Method for Measuring Content of Prasugrel and CATP in Prasugrel Hydrochloride)

The contents of prasugrel and CATP in prasugrel hydrochloride was measured as described below.

In an acetonitrile-water mixed solution (7:3) was dissolved 150 mg of prasugrel hydrochloride to 100 mL. Under the following conditions, 10 µL of the solution was subjected to liquid chromatography for measurement.
Measurement conditions (liquid chromatography)
Detector: ultraviolet absorptiometer (detection wavelength: 240 nm)
Analytical Column: Cadenza CD-C18, inner diameter; 4.6 mm, length; 15 cm, particle size; 3 µm
Guard column: none
Column temperature: 40°C
Mobile phase: 0.01 mol/L potassium dihydrogenphosphate aqueous solution:tetrahydrofuran:acetonitrile = 13:5:2 (V/V/V)
Flow rate: 1.0 mL/min.

**Table 1**

| (Content of CATP in Prasugrel Hydrochloride) | |
|---|---|
| Content (%) of CATP in Prasugrel Hydrochloride | |
| Example 1 | 0.031 |
| Example 2 | 0.014 |
| Reference Example 1 | 0.042 |

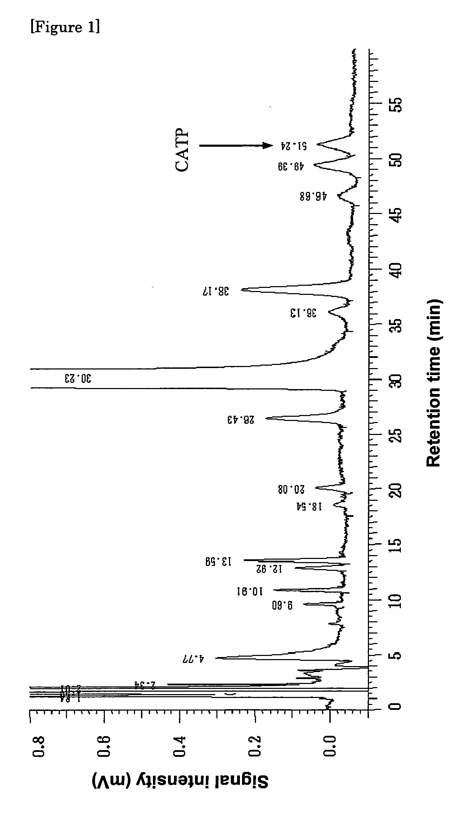

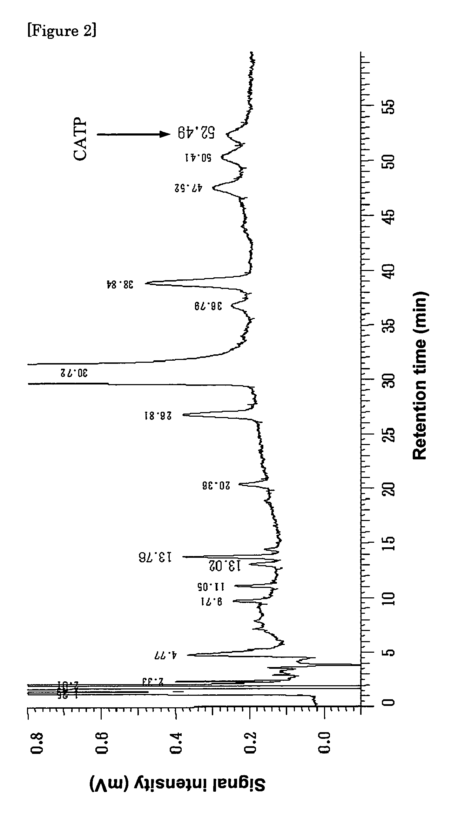

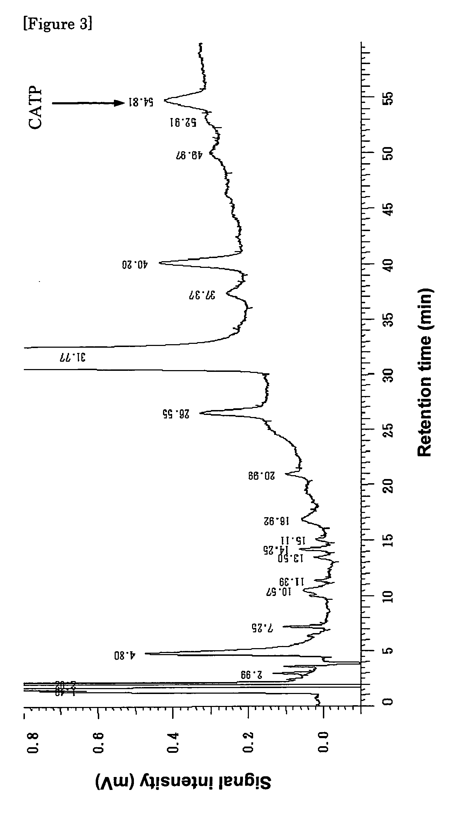

The content of prasugrel and CATP are expressed in percentage by area (%) measured using the above liquid chromatography. The results of liquid chromatography of the prasugrel hydrochloride obtained in Examples 1 and 2 and Reference Example 1 are shown in Figures 1, 2 and 3, respectively.

The content of CATP in the final product prasugrel hydrochloride was distinctly lower in Examples 1 and 2, in which the addition of chlorine gas and the reaction thereafter in step (i) were performed at low temperature, than in Reference Example 1 in which the reaction after addition of chlorine gas was carried out at room temperature. The content of CATP in the final product prasugrel hydrochloride was also reduced more in Example 2, in which 2-(tert-butyldimethylsilyloxy)-5-(α-cyclopropylcarbonyl-2-fluorobenzyl)-4,5,6,7-tetrahydrothieno[3,2-c]pyridine obtained in step (ii) was purified by recrystallization, than in Example 1 in which the recrystallization was not performed.

### Industrial Applicability

According to the present invention,a method for producing high-purity prasugrel hydrochloride with a reduced content of impurities such as the by-product CATP is obtained.

## Claims

1. A method for producing prasugrel hydrochloride, comprising the steps of:
(i) chlorinating a compound represented by the formula: 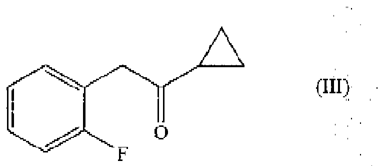 by adding a chlorinating agent thereto in a solvent;
(ii) reacting the resultant compound represented by the formula: 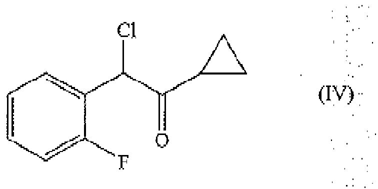 with a compound represented by the general formula: 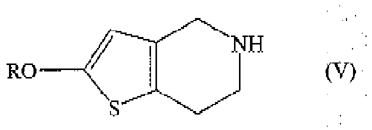 wherein R represents a protecting group for a hydroxyl group,
or a salt thereof in a solvent in the presence of a base;
(iii) acetylating the resultant compound represented by the general formula: 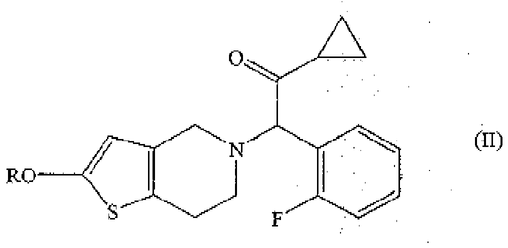 wherein R has the same meaning as above,
by reacting an acetylating agent therewith in a solvent in the presence of a base and an acylation catalyst, and
(iv) adding hydrochloric acid to the resultant compound represented by the formula: 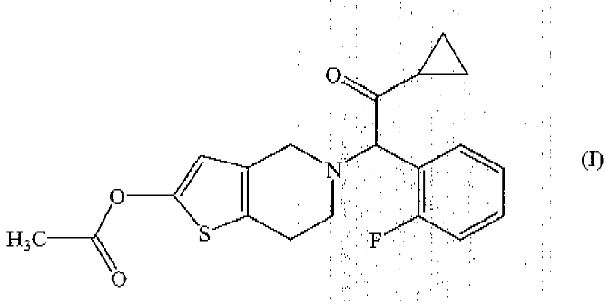 in a solvent, thereby producing prasugrel hydrochloride represented by the formula: 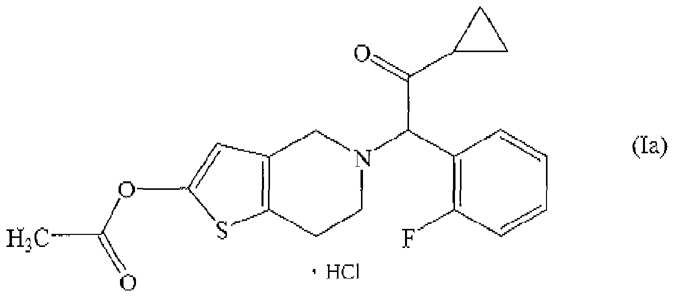 **characterized in that**, in step (i), the temperature during the addition of the chlorinating agent is -20°C to 5°C and the reaction temperature after the addition of the chlorinating agent is -20°C to 5°C.

2. A method according to claim 1, **characterized in that**, in step (i), the temperature during the addition of the chlorinating agent is -10°C to 5°C and the reaction temperature after the addition of the chlorinating agent is -10°C to 5°C.

3. A method according to claim 1, **characterized in that**, in step (i), the temperature during the addition of the chlorinating agent is -5°C to 5°C and the reaction temperature after the addition of the chlorinating agent is -5°C to 5°C.

4. A method according to any one of claims 1 to 3, **characterized in that** the chlorinating agent is added dropwise.

5. A method according to any one of claims 1 to 4, **characterized in that** the hydrochloric acid is added dropwise.

6. A method according to any one of claims 1 to 5, **characterized in that** the temperature of post-treatment after the end of the reaction in step (i) is -20°C to 15°C.

7. A method according to any one of claims 1 to 5, **characterized in that** the temperature of post-treatment after the end of the reaction in step (i) is -10°C to 15°C.

8. A method according to any one of claims 1 to 5, **characterized in that** the temperature of post-treatment after the end of the reaction in step (i) is 0°C to 15°C.

9. A method according to any one of claims 1 to 5, wherein the chlorinating agent is chlorine gas.

10. A method according to any one of claims 1 to 9, wherein R is a group represented by the general formula: 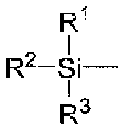 wherein R¹, R², and R³ independently represent am alkyl group having 1 to 10 carbons or an aryl group.

11. A method according to claim 10, wherein R¹ R² and R³ independently represent an alkyl group having 1 to 5 carbons or a phenyl group.

12. A method according to any one of claims 1 to 9, wherein R is a tert-butyldimethylsilyl group.

13. A method according to any one of claims 1 to 12, **characterized in that** the resultant compound represented by the general formula (II) is recrystallized from others or nitriles in step (ii),

14. A method according to any on of claims 1 to 12, **characterized in that** the resultant compound represented by the general formula (II) is recrystallized from acetonitrile in step (ii).

15. A method according to any one of claims 1 to 14, wherein the acetylating agent is acetic anhydride.

16. A method according to any one of claims 1 to 15, **characterized in that** the resultant compound represented by the formula (I) obtained in step (iii) is used in the next step (iv) without purification.

## Patentansprüche

1. Verfahren zur Herstellung von Prasugrelhydrochlorid, umfassend die folgenden Schritte:
(i) Chlorieren einer Verbindung, die durch die folgende Formel dargestellt wird: 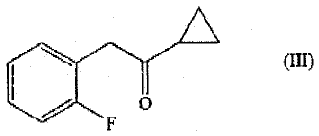 durch Zugabe eines Chlorierungsmittels in einem Lösungsmittel;
(ii) Reagierenlassen der entstehenden, durch die folgende Formel dargestellten Verbindung: 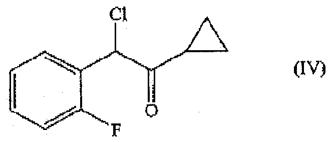 mit einer durch die folgende allgemeine Formel dargestellten Verbindung: 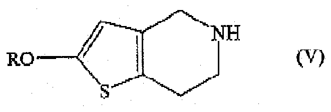 wobei R eine Schutzgruppe für eine Hydroxylgruppe repräsentiert,
oder einem Salz davon in einem Lösungsmittel in Anwesenheit einer Base;
(iii) Acetylieren der entstehenden, durch die folgende allgemeine Formel dargestellten Verbindung 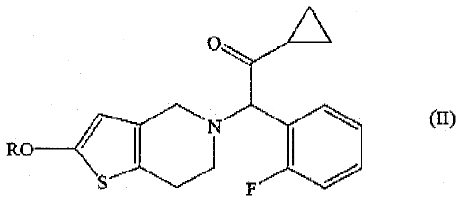 wobei R die gleiche Bedeutung wie oben hat,
indem ein Acetylierungsmittel in einem Lösungsmittel in Anwesenheit einer Base und eines Acetylierungskatalysators damit zur Reaktion gebracht wird; und
(iv) Zugabe von Chlorwasserstoffsäure zu der entstehenden, durch die folgende Formel dargestellten Verbindung: 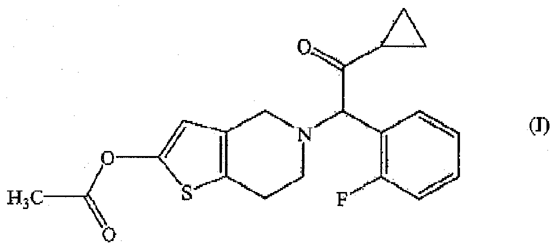 in einem Lösungsmittel, dadurch Erzeugen von Prasugrelhydrochlorid, das durch die folgende Formel dargestellt wird: 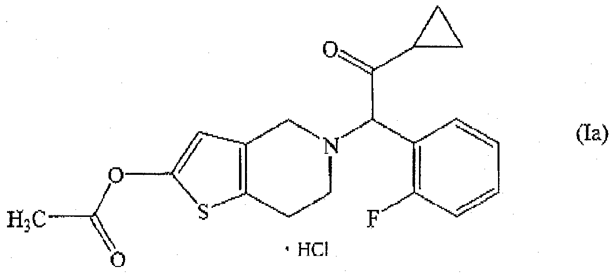 **dadurch gekennzeichnet, dass** im Schritt (i) die Temperatur während der Zugabe des Chlorierungsmittels -20°C bis 5°C beträgt und die Reaktionstemperatur nach der Zugabe des Chlorierungsmittels -20°C bis 5°C beträgt.

2. Verfahren nach Anspruch 1, **dadurch gekennzeichnet, dass** im Schritt (i) die Temperatur während der Zugabe des Chlorierungsmittels -10°C bis 5°C beträgt und die Reaktionstemperatur nach der Zugabe des Chlorierungsmittels -10°C bis 5°C beträgt.

3. Verfahren nach Anspruch 1, **dadurch gekennzeichnet, dass** im Schritt (i) die Temperatur während der Zugabe des Chlorierungsmittels -5°C bis 5°C beträgt und die Reaktionstemperatur nach der Zugabe des Chlorierungsmittels -5°C bis 5°C beträgt.

4. Verfahren nach einem der Ansprüche 1 bis 3, **dadurch gekennzeichnet, dass** das Chlorierungsmittel tropfenweise zugesetzt wird.

5. Verfahren nach einem der Ansprüche 1 bis 4, **dadurch gekennzeichnet, dass** die Chlorwasserstoffsäure tropfenweise zugesetzt wird.

6. Verfahren nach einem der Ansprüche 1 bis 5, **dadurch gekennzeichnet, dass** die Temperatur der Nachbehandlung nach dem Ende der Reaktion in Schritt (i) -20°C bis 15°C beträgt.

7. Verfahren nach einem der Ansprüche 1 bis 5, **dadurch gekennzeichnet, dass** die Temperatur der Nachbehandlung nach dem Ende der Reaktion in Schritt (i) -10°C bis 15°C beträgt.

8. Verfahren nach einem der Ansprüche 1 bis 5, **dadurch gekennzeichnet, dass** die Temperatur der Nachbehandlung nach dem Ende der Reaktion in Schritt (i) 0°C bis 15°C beträgt.

9. Verfahren nach einem der Ansprüche 1 bis 5, wobei das Chlorierungsmittel Chlorgas ist.

10. Verfahren nach einem der Ansprüche 1 bis 9, wobei R eine durch die folgende allgemeine Formel dargestellte Gruppe ist: 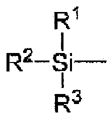 wobei R¹, R² und R³ unabhängig voneinander eine Alkylgruppe mit 1 bis 10 Kohlenstoffatomen oder eine Arylgruppe darstellen.

11. Verfahren nach Anspruch 10, wobei R¹, R² und R³ unabhängig voneinander eine Alkylgruppe mit 1 bis 5 Kohlenstoffatomen oder eine Phenylgruppe darstellen.

12. Verfahren nach einem der Ansprüche 1 bis 9, wobei R eine tert-Butyldimethylsilylgruppe ist.

13. Verfahren nach einem der Ansprüche 1 bis 12, **dadurch gekennzeichnet, dass** die durch die allgemeine Formel (II) dargestellte resultierende Verbindung im Schritt (ii) aus Ethern oder Nitrilen umkristallisiert wird.

14. Verfahren nach einem der Ansprüche 1 bis 12, **dadurch gekennzeichnet, dass** die durch die allgemeine Formel (II) dargestellte resultierende Verbindung im Schritt (ii) aus Acetonitril umkristallisiert wird.

15. Verfahren nach einem der Ansprüche 1 bis 14, wobei das Acetylierungsmittel Essigsäureanhydrid ist.

16. Verfahren nach einem der Ansprüche 1 bis 15, **dadurch gekennzeichnet, dass** die in Schritt (iii) gewonnene, durch die Formel (I) dargestellte resultierende Verbindung im nächsten Schritt (iv) ohne Reinigung eingesetzt wird.

## Revendications

1. Procédé pour produire du chlorhydrate de prasugrel, comprenant les étapes consistant:
(i) à chlorer un composé représenté par la formule: 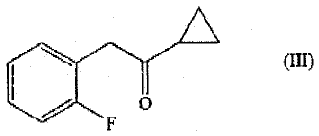 en ajoutant un agent chlorant à celui-ci dans un solvant;
(ii) à faire réagir le composé obtenu représenté par la formule: 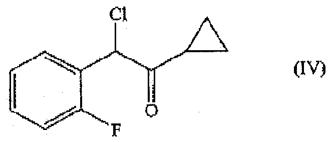 avec un composé représenté par la formule générale: 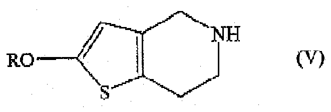 dans laquelle R représente un groupe protecteur pour un groupe hydroxyle, ou un sel de celui-ci dans un solvant en présence d'une base;
(iii) à acétyler le composé obtenu représenté par la formule générale: 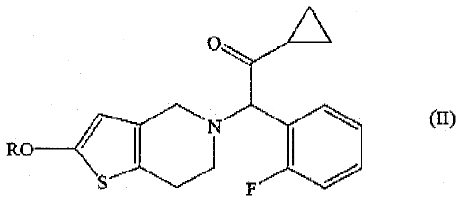 dans laquelle R a la même signification que ci-dessus,
en faisant réagir un agent acétylant avec celui-ci dans un solvant en présence d'une base et d'un catalyseur d'acylation; et
(iv) à ajouter de l'acide chlorhydrique au composé obtenu représenté par la formule: 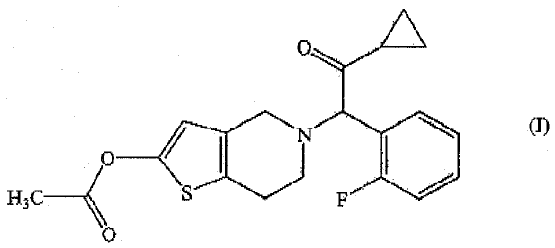 dans un solvant, produisant ainsi un chlorhydrate de prasugrel représenté par la formule: 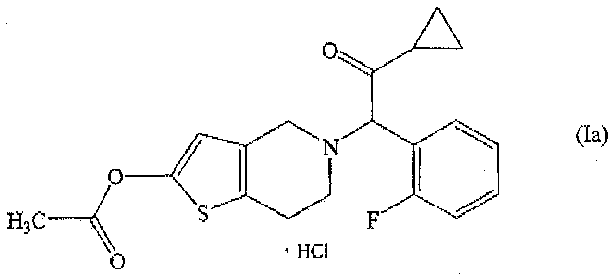 **caractérisé en ce que**, dans l'étape (i), la température pendant l'addition de l'agent chlorant est de -20°C à 5°C et la température de réaction après l'addition de l'agent chlorant est de -20°C à 5°C.

2. Procédé selon la revendication 1, **caractérisé en ce que**, dans l'étape (i), la température pendant l'addition de l'agent chlorant est de -10°C à 5°C et la température de réaction après l'addition de l'agent de chlorant est de -10°C à 5°C.

3. Procédé selon la revendication 1, **caractérisé en ce que**, dans l'étape (i), la température pendant l'addition de l'agent chlorant est de -5°C à 5°C et la température de réaction après l'addition de l'agent chlorant est de -5°C à 5°C.

4. Procédé selon l'une quelconque des revendications 1 à 3, **caractérisé en ce que** l'agent chlorant est ajouté goutte à goutte.

5. Procédé selon l'une quelconque des revendications 1 à 4, **caractérisé en ce que** l'acide chlorhydrique est ajouté goutte à goutte.

6. Procédé selon l'une quelconque des revendications 1 à 5, **caractérisé en ce que** la température du post-traitement après la fin de la réaction dans l'étape (i) est de -20°C à 15°C.

7. Procédé selon l'une quelconque des revendications 1 à 5, **caractérisé en ce que** la température du post-traitement après la fin de la réaction dans l'étape (i) est de -10°C à 15°C.

8. Procédé selon l'une quelconque des revendications 1 à 5, **caractérisé en ce que** la température du post-traitement après la fin de la réaction dans l'étape (i) est de 0°C à 15°C.

9. Procédé selon l'une quelconque des revendications 1 à 5, dans lequel l'agent chlorant est du chlore gazeux.

10. Procédé selon l'une quelconque des revendications 1 à 9, dans lequel R est un groupe représenté par la formule générale: 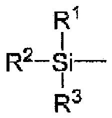 dans laquelle R¹, R² et R³ représentent indépendamment un groupe alkyle ayant 1 à 10 carbones ou un groupe aryle.

11. Procédé selon la revendication 10, dans lequel R¹, R² et R³ représentent indépendamment un groupe alkyle ayant 1 à 5 carbones ou un groupe phényle.

12. Procédé selon l'une quelconque des revendications 1 à 9, dans lequel R est un groupe tert-butyldiméthylsilyle.

13. Procédé selon l'une quelconque des revendications 1 à 12, **caractérisé en ce que** le composé obtenu représenté par la formule générale (II) est recristallisé à partir d'éthers ou de nitriles dans l'étape (ii).

14. Procédé selon l'une quelconque des revendications 1 à 12, **caractérisé en ce que** le composé obtenu représenté par la formule générale (II) est recristallisé à partir d'acétonitrile dans l'étape (ii).

15. Procédé selon l'une quelconque des revendications 1 à 14, dans lequel l'agent acétylant est l'anhydride acétique.

16. Procédé selon l'une quelconque des revendications 1 à 15, **caractérisé en ce que** le composé obtenu représenté par la formule (I) obtenu dans l'étape (iii) est utilisé dans l'étape (iv) suivante sans purification.
